# EUROPEAN PATENT APPLICATION

(11) **EP 4 335 354 A1**
(43) Date of publication of application: **13.03.2024**
(21) Application number: 22798693.2
(22) Date of filing: 01.04.2022
(51) Int. Cl.: A61B 3/10

(54) **DEVICE FOR DETERMINING THE CENTRING OF CONTACT OR INTRAOCULAR LENSES**

(30) Priority: 07.05.2021 ES 202130936 U
(71) Applicant: Sport Vision S.L., 09550 Villarcayo Burgos (ES)
(72) Inventor: CALVO LEÓN, Juan Carlos, 48980 SANTURTZI BIZKAIA (ES); VILLAVERDE ROSENDE, Julio, 09500 MEDINA DE POMAR - BURGOS (ES)
(74) Representative: Del Valle Valiente, Sonia
(86) International application number: PCT/ES2022/070196
(87) International publication number: WO 2022/234164

(57) **Abstract**

The invention relates to a device (1) for determining the centring of contact or intraocular lenses, which comprises a concentrated light generator (3) for generating a well-defined first binocular Purkinje image (2), an aligner of the patient with the light generator (3), a distance meter (5) for measuring the distance between the light generator and the patient (8) during the operation of the device (1), and a position meter (6) for measuring the position of the first binocular Purkinje image (2) generated in the eyes (7) of the patient (8).

## Description

### OBJECT OF THE INVENTION

The present invention relates to a device for determining the centring of contact or intraocular lenses through the position of the first Purkinje image.

### BACKGROUND OF THE INVENTION

Currently, the use of variable focus and progressive lenses has the advantage that, with a single lens, it allows the patient to satisfactorily see at different distances, despite the vision deficiencies of the eye, which may be for short and long distances for the same patient. For this reason, progressive lenses are designed with an area adapted to far vision and another to near vision, and they are provided with an intermediate zone between the optical centre for far vision and the optical centre for near vision, which changes gradually, allowing it to adapt to different intermediate distances. The horizontal distance between the far and near optical centres is known as inset, while the vertical distance is known as segment drop.

To achieve this satisfactory vision with progressive lenses, it is essential for the optometrist to perform not only a good dioptre measurement, but also the most functionally correct determination of the optical centre of said lens.

In the known art, the optical centre is determined in two main ways: through standard pre-established positions, or through specific measurements. The first case, although it has advantages such as the mass production of lenses, is not very adaptable for some users, especially in the event that there are facial asymmetries or other types of conditions that deviate the location of the optical centre with respect to said standard position. In the second case, the usual methods start from observing the location of the pupils of the user when they look at an object located at the vision distance for which the lenses are designed. In this case, by projecting the imaginary line that joins the object with the centres of each retina, this line crosses the geometric axis of the eye and the point at which it would cross the lens can be determined, once placed in the position of use thereof in glasses. This becomes complicated when several optical centres must be measured, as in the case of multifocal and progressive lenses, and success depends greatly on the experience and skill of the optometrist. Even with this, it is not uncommon that the determination of the optical centres of the lenses may not totally coincide with the real axes of vision of the user. Thus, the lens is displaced with respect to the position that the user would really need. In cases where the deviation is not very large, the user can adjust their vision, although side effects such as headaches or eye strain may arise. In more severe cases, binocular vision may be lost, vision may be blurred, etc.

To solve this problem, the applicant is the holder of patent P 201730494 relating to a design method for a pair of ophthalmic lenses, one for each eye, comprising a measurement step where a vision distance is determined and a reference object is placed at a point located at said vision distance; a reference frame is placed on the patient, configured to determine a wearing position of the lenses; a screen provided with a hole is successively placed on each eye of the patient and the position of the hole is moved until the patient sees the reference object with both eyes at the same time (binocular vision), and the position of the holes is measured with respect to the position of use of the lenses that are being designed.

To this end, this patent also describes a device comprising a frame fixable in front of the eyes of the patient, two non-transparent screens (one per eye), each configured by parallel plates with crossed slots and slidably mounted respectively in the vertical and horizontal direction, to define the hole on each eye in the area where they cross, and to be able to move the position thereof simply by sliding both plates of each screen.

This method, and especially the configuration of the device, works satisfactorily when it comes to designing frame lenses, that is, those that are going to be mounted on glasses, but it does not work so satisfactorily when it comes to designing ocular lenses, whether contact or intraocular lenses, which is a problem that is solved with the device of the invention.

### DESCRIPTION OF THE INVENTION

The device for determining the centring of contact or intraocular lenses of the invention comprises, in its most generic embodiment:
- a concentrated light generator (that is spot, annular, etc.) to generate a first binocular Purkinje image (in both eyes of the patient at the same time) that is equally well concentrated or defined (not diffuse),
- an aligner of the patient with the light generator to place the light generator coaxially with the binocular vision of the patient,

- a distance meter for measuring the distance between the light generator and the patient during the operation of the device, and
- a position meter of the first binocular Purkinje image generated in the eyes of the patient.

In this way, the position meter of the first Purkinje image measures the deviation in two axes (horizontal and vertical) of said image with respect to the geometric axis of the eye, to determine the optical centre at the chosen distance. This can be done, most preferably, through a camera and an image processor with a specific program.

But it also has the following advantages from an optical point of view:
- Better visual quality.
- Less prismatic effect.
- Minimal transverse chromatic aberration.
- Better binocular balance.
- Decrease in spherical aberration.
- More precise optical correction.
- Better foveolar centring.
- More aplanatic optical system.

This also provides the following advantages to the patient:
- Better visual comfort because this is directly related to centring.
- The phenomena of parasitic lights are reduced.
- Vertical misalignments of the visual axes can be more easily compensated, which cause a multitude of musculoskeletal problems and work absenteeism, due to postural pain, related to these alterations.
- Greatly improves binocular visual convergence at short visual distances.
- Minimises interventions (operations) for correction or rejection of intraocular lenses due to non-adaptation to vision.

### DESCRIPTION OF THE DRAWINGS

Figure 1 shows a view of the device in operation determining the centring contact or intraocular lenses of a patient.
Figure 2 shows a view of a support provided with fastening means for fastening to glasses, in which the straight spot beam laser generator, the laser distance meter, and the graphical references of known size and position are mounted for photogrammetry.

### PREFERRED EMBODIMENT OF THE INVENTION

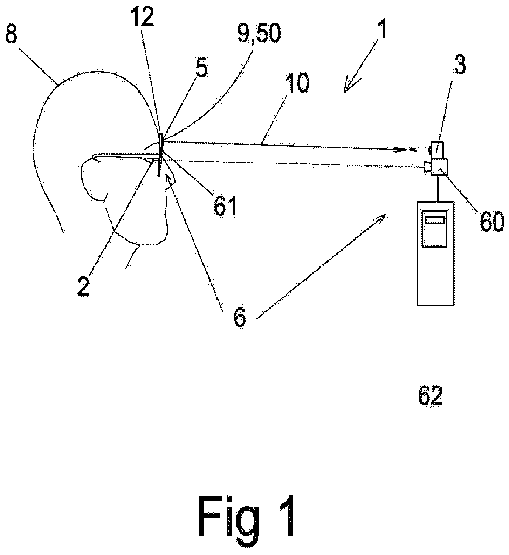

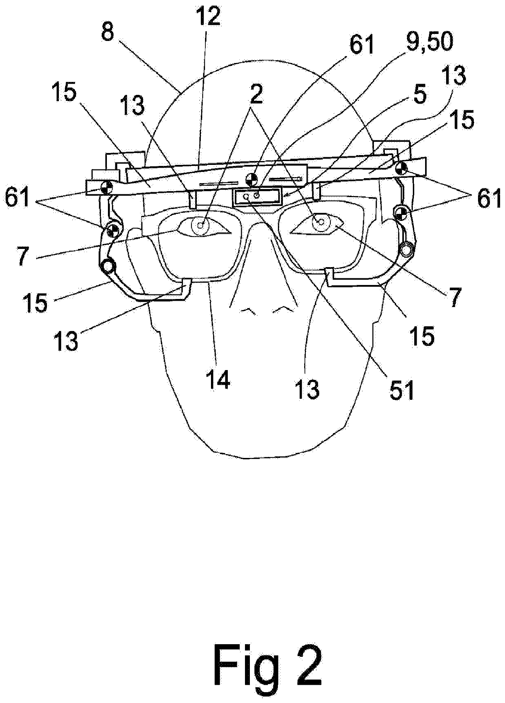

The device (1) (see fig. 1) for determining the centring of contact or intraocular lenses (see also fig. 2) of the invention comprises (return to fig. 1):
- a concentrated light generator (3) for generating a first binocular Purkinje image (2), in both eyes of the patient at the same time as seen in fig. 2,
- an aligner of the patient with the light generator to place the light generator coaxially with the binocular vision of the patient,
- a distance meter (5) for measuring the distance between the light generator and the patient (8) during the operation of the device (1), and
- a position meter (6) of the first binocular Purkinje image (2) generated in the eyes (7) of the patient (8).

In the embodiment shown in the figures, the position meter (6) of the first binocular Purkinje image (2) comprises (see fig. 1) a capture element (60) of said first Purkinje image (2) -in each eye-, graphical references (61) (see fig. 2) of known size and position, and (return to fig. 1) an image processor (62) using photogrammetry, to measure the position of the Purkinje images with respect to the geometric axis of the eye, ideally in the vertical and horizontal axes. This, thanks to photogrammetry and graphical references, allows the pupil distance data to be obtained in both axes directly in digital format, although the position measurement could also be performed by other, manual, means for example.

As for the aligner of the patient with the spot light generator, said aligner preferably comprises a laser generator (9) with a straight spot beam (10), arranged medially between the eyes (7) of the patient (8), as seen in fig. 2, and the beam (10) of which is oriented in an outward direction perpendicularly from the face of the patient (8) as seen in fig. 1. In this way, the patient (8) during the tasks points the laser beam (10) at the concentrated or spot light generator (3), and where they both line up determines the coaxiality.

Furthermore, it is also preferred that the distance meter (5) for measuring the distance between the light generator (3) and the patient (8) comprises a laser rangefinder (which comprises a laser emitter (50) and a sensor (51) - see fig. 2-to capture the reflection of the laser light emitted by said laser emitter in the light generator (3)). This has the advantage that the laser generator (9) with a straight spot beam (10) can be configured at the same time by the laser emitter (50) of the laser rangefinder, as seen in the preferred embodiment represented in the figures.

Lastly, it must be noted that the device ideally comprises a support (12) in the form of glasses, or provided with fastening means for fastening to glasses, on which are mounted:
- the laser generator (9) with a straight spot beam (10),
- the laser distance meter (5) (rangefinder), and
- the graphical references (61) of known size and position for photogrammetry.

Said support (12) may comprise fastening grips (13) (see fig. 2) for fastening to the frame of glasses (14), and extendable or articulated arms (15) on which some of the grips (13) are arranged, to adapt to the shape of the frame of any glasses.

Having sufficiently described the nature of the invention, it is noted that the description thereof and its preferred embodiment must be interpreted in a nonlimiting manner, and that it covers all the possible embodiment variants that can be deduced from the content of this specification and the claims.

## Claims

1. A device (1) for determining the centring of contact or intraocular lenses **characterised in that** it comprises:
- a concentrated light generator (3) for generating a well-defined first binocular Purkinje image (2),
- an aligner of the patient with the light generator (3),
- a distance meter (5) for measuring the distance between the light generator and the patient (8) during the operation of the device (1), and
- a position meter (6) of the first binocular Purkinje image (2) generated in the eyes (7) of the patient (8).

2. The device (1) for determining the centring of contact or intraocular lenses according to claim 1, **wherein** the position meter (6) of the first binocular Purkinje image (2) comprises a capture element (60) of said first Purkinje image (2), graphical references (61) of known size and position, and an image processor (62) using photogrammetry.

3. The device (1) for determining the centring of contact or intraocular lenses according to any of the preceding claims, **wherein** the aligner of the patient with the spot light generator comprises a laser generator (9) with a spot beam (10), arranged medially between the eyes (7) of the patient (8) and the beam (10) of which is oriented perpendicularly in an outward direction perpendicularly from the face of the patient (8).

4. The device (1) for determining the centring of contact or intraocular lenses according to any of the preceding claims, **wherein** the distance meter (5) for measuring the distance between the light generator (3) and the patient (8) comprises a laser rangefinder.

5. The device (1) for determining the centring of contact or intraocular lenses according to preceding claims, **wherein** the laser generator (9) with a straight spot beam (10) is configured by the laser emitter (50) of the laser rangefinder.

6. The device (1) for determining the centring of contact or intraocular lenses according to preceding claims, **that** comprises a support (12) in the form of glasses, or provided with fastening means for fastening glasses, on which are mounted:
- the laser generator (9) with a spot beam (10),
- the laser distance meter (5), and
- the graphical references (61) of known size and position for photogrammetry.

7. The device (1) for determining the centring of contact or intraocular lenses according to claim 6, **wherein** the support (12) comprises fastening grips (13) for fastening to the frame of glasses (14).

8. The device (1) for determining the centring of contact or intraocular lenses according to claim 7, **wherein** the support (12) comprises extendable or articulated arms (15) on which some of the grips (13) are arranged.
